# EUROPÄISCHE PATENTANMELDUNG

(11) **EP 3 360 518 A1**
(43) Veröffentlichungstag der Anmeldung: **15.08.2018**
(21) Anmeldenummer: 18150289.9
(22) Anmeldetag: 04.01.2018
(51) Int. Cl.: A61F 7/08

(54) **WÄRMFLASCHE**

(30) Priorität: 08.02.2017 DE 202017100657 U
(71) Anmelder: Sejfuli, Florentina, 47053 Duisburg (DE)
(72) Erfinder: Sejfuli, Florentina, 47053 Duisburg (DE)
(74) Vertreter: Nobbe, Matthias

(57) **Zusammenfassung**

Die Erfindung betrifft eine Wärmflasche mit einem Flaschenkörper mit einer Zentralkammer zur Aufnahme eines Wärmeübertragungsmediums und mit vier schlauchförmigen Kammern, die jeweils am Flaschenkörper angesetzt in Fluidverbindung mit der Zentralkammer im Flaschenkörper stehen und die an dem der Zentralkammer entgegengesetzten Ende verschlossen sind.

## Beschreibung

Die Erfindung betrifft eine Wärmflasche mit einem Flaschenkörper mit einer Zentralkammer zur Aufnahme eines Wärmeübertragungsmediums und mit vier schlauchförmigen Kammern, die jeweils am Flaschenkörper angesetzt in Fluidverbindung mit der Zentralkammer im Flaschenkörper stehen und die an dem der Zentralkammer entgegengesetzten Ende verschlossen sind.

Wärmflaschen werden zur Wärme- aber auch Kältetherapie eingesetzt. Sie bestehen zumeist aus einer flexiblen, hitzebeständigen Kunststoffkammer und sind mit einer Einfüll- beziehungsweise Entleeröffnung versehen, durch die die Wärmflasche vor Behandlungsbeginn mit Wasser befüllt werden kann. Diese Wärmflaschen weisen in der Draufsicht vorwiegend die Form eines Rechtecks mit abgerundeten Ecken auf.

Nachteilig bei den aus dem Stand der Technik bekannten Wärmflaschen ist, dass die wärme- oder kälteübertragende Fläche der Wärmflasche oftmals nicht mit der Fläche der zu behandelnden Körperpartie übereinstimmt. Daher sind die aus dem Stand der Technik bekannten Wärmflaschen für bestimmte Körperpartien oftmals entweder zu groß oder zu klein.

Die Aufgabe der Erfindung ist es, eine Wärmflasche bereit zu stellen, die die Nachteile aus dem Stand der Technik überwindet. Somit ist die Aufgabe, eine Wärmflasche bereitzustellen, die sich für die Behandlung von unterschiedlich großen Körperpartien eignet.

Zur Lösung der Aufgabe ist eine Wärmflasche mit einem Flaschenkörper mit einer Zentralkammer zur Aufnahme eines Wärmeübertragungsmediums und mit vier schlauchförmigen Kammern, die jeweils am Flaschenkörper über einen Verbindungsbereich angesetzt in Fluidverbindung mit der Zentralkammer im Flaschenkörper stehen und die an dem der Zentralkammer entgegengesetzten Ende verschlossen sind, wobei die Wärmflasche in der Draufsicht die Form eines Kreuzes hat, wobei an zumindest zwei von Flaschenkörper und Kammern Befestigungsmittel angeordnet sind, vorgesehen.

Die mit der Erfindung erzielten Vorteile bestehen unter anderem darin, dass die Größe der Wärme- oder Kälteübertragungsfläche der erfindungsgemäßen Wärmflasche variabel einstellbar ist, sodass die Wärmflasche zur Behandlung von unterschiedlich großen Körperpartien geeignet ist. Die Wärmeübertragungsfläche kann durch Umklappen der schlauchförmigen Kammern auf den Flaschenkörper verkleinert werden. Für den Fall, dass eine größere Körperpartie behandelt werden soll, wird die Wärmflasche in der teilweise ausgebreiteten Form oder in der vollständig ausgebreiteten Form verwendet. Bei einer solchen Anwendungsweise strahlt die Wärme auch auf die Körperbereiche aus, die sich seitlich zwischen den Kammern befinden und nicht direkt von der Wärmflasche bedeckt sind. Die Befestigungsmittel ermöglichen eine Befestigung aneinander und/oder eine Befestigung an der zu behandelnden Objekt.

Die Zentralkammer des Flaschenkörpers steht mit den schlauchförmigen Kammern in Fluidverbindung, sodass sich das Wärmeübertragungsmedium in der gesamten Wärmflasche verteilen kann.

Erfindungsgemäß ist unter Wärmeübertragungsmedium ein Medium zu verstehen, das entweder eine höhere Temperatur als die zu behandelnde Körperpartie aufweist, sodass Wärme von dem Wärmeübertragungsmedium auf die Körperpartie übertragen wird, oder das eine geringere Temperatur als die zu behandelnde Körperpartie aufweist, sodass Wärme von der Körperpartie auf das Wärmeübertragungsmediums übertragen wird. Demzufolge ist unter Wärmeübertragungsmedium ebenso ein Kälteübertragungsmedium zu verstehen. Demzufolge ist unter der erfindungsgemäßen Wärmflasche ebenso ein "Kühlpack" zu verstehen.

Als Wärmeübertragungsmedien können Wasser, Getreidekörner, Obstkerne, unterkühlte Schmelzen, insbesondere in Form von Natriumacetat-Trihydrat, oder elektrische Heiz- oder Kühlelemente, gewünschtenfalls auch in Kombination mit einem der zuvor genannten Medien, verwendet werden. Eine solche Wärmflasche kann unter anderem durch ein Wasserbad, durch Erhitzen in der Mikrowelle, Kühlen in Kühl- oder Gefrierschränken oder Anlegen von Spannung auf die gewünschte Temperatur gebracht werden. Wird eine unterkühlte Schmelze als Wärmeübertragungsmedium, beispielsweise von Natriumacetat-Trihydrat, verwendet, kann der metastabile Zustand beispielsweise durch das Knicken eines Metallplättchens, das sich in der unterkühlten Schmelz befindet, beendet und die chemische, exotherme Reaktion in Gang gesetzt werden.

Als Wärmflaschenmaterial kann unter anderem das aus dem Stand der Technik bekannte flexible, hitzebeständige Kunststoffmaterial verwendet werden. Ebenso ist es möglich, dass die Wärmflasche textiles Material aufweist. In einer weiteren Ausführungsform ist eine Hybridwärmflasche vorgesehen, die sowohl Kunststoffmaterial als auch textiles Material umfasst.

Jede der schlauchförmigen Kammern kann in der Draufsicht von oben die Form eines Rechtecks, die Form eines Rechtecks mit abgerundeten Ecken an dem den Flaschenkörper entgegengesetzten Ende, die Form eines Rechteckes mit angesetztem Halbkreis, die Form eines Halbkreises oder eine birnenförmige Form aufweisen Dabei können die einzelnen Kammern eine gleiche oder eine unterschiedliche Form in der Draufsicht von oben haben. In einer Ausführungsform haben zumindest zwei gegenüberliegende Kammern die gleiche Form.

In der Draufsicht hat die Wärmflasche erfindungsgemäß die Form eines Kreuzes, d.h. die Winkel zwischen den Kanten von zwei benachbarten Kammern betragen in etwa 90°.

In einer vorteilhaften Ausgestaltung der Erfindung sind die Befestigungsmittel zur Befestigung aneinander ausgebildet, beispielsweise nach Art eines Klettverschlusses. Dies ermöglicht beispielsweise, dass die auf den Flaschenkörper geklappten Kammern an diesem mittels der Befestigungsmittel befestigt werden können, sodass ein ungewolltes Zurückklappen verhindert wird.

In einer vorteilhaften Ausgestaltung der Erfindung sind die Befestigungsmittel zur Befestigung der Wärmflasche an dem zu wärmenden Objekt ausgebildet. Somit kann die Wärmflasche beispielsweise um ein Körperteil, zum Beispiel ein Bein, oder eine Kleidungsstück gelegt werden, wobei die zwei das Köperteil bzw. Kleidungsstück umgebende Kammern mittels der Befestigungsmittel aneinander befestigt werden, um auf diese Weise die Wärmflasche an das Körperteil oder das Kleidungsstück zu fixieren. Im Falle einer Blasenentzündung beispielsweise kann die Wärmflasche so getragen werden, dass eine Kammer die Vorderseite und die gegenüberliegende Kammer die Rückseite des Anwenders erwärmt, während die zwei anderen Kammern, die die Unterkleidung des Anwenders umschließen, aneinander befestigt werden und so die Wärmflasche an der Unterkleidung fixieren.

Bevorzugt sind die Befestigungsmittel zur Befestigung der Wärmflasche an Textilien ausbildet. Um beispielsweise ein Verrutschen durch Körperbewegungen zu minimieren, kann die Wärmflasche derartige Befestigungsmittel aufweisen. Dies ermöglicht eine Fixierung unterhalb eines Kleidungsstückes, ohne dass die Wärmflasche verrutscht.

Vorzugsweise sind Befestigungsmittel am Flaschenkörper und an jeder Kammer angeordnet.

In einer bevorzugten Ausgestaltung der Erfindung umfasst die erfindungsgemäße Wärmflasche zusätzlich ein Verlängerungselement, das über zumindest eines der Befestigungsmittel mit dem Flaschenkörper oder einer der Kammern verbunden ist. Bevorzugt ist das Verlängerungselement über zwei Befestigungsmittel mit dem Flaschenkörper oder den Kammern verbunden. Ganz besonders bevorzugt ist das Verlängerungselement über Befestigungsmittel mit zwei sich dem Flaschenkörper gegenüber angeordneten Kammern verbunden. Vorzugsweise umfasst das Verlängerungselement eine elastische Komponente, insbesondere ein elastisches Band. In der Ausführungsform mit einem Verlängerungselement wird ein Anbringen der Wärmflasche um ein Körperteil wie um einen Arm oder ein Bein oder auch den Rumpf erleichtert.

In einer vorteilhaften Ausgestaltung der Erfindung sind der Flaschenkörper und die vier schlauchförmigen Kammern jeweils als Flachkörper ausgebildet. Unter einem Flachkörper wird erfindungsgemäß ein Körper verstanden, dessen Ausbreitung in z-Richtung geringer ist als die in der Draufsicht erkennbare Ausbreitung in der x- und y-Ebene. Ein solcher Flachkörper ermöglicht die Behandlung einer großen Körperfläche bei geringem Eigengewicht. Zudem wird durch die geringe Dicke ein Umklappen jeder der schlauchförmigen Kammern auf den Flaschenkörper erleichtert und der Flaschenkörper trägt nicht so auf, wenn er beispielsweise vom Benutzer unter der Kleidung getragen wird.

Es ist ebenso möglich, dass die einzelnen Kammern eine unterschiedliche Dicke aufweisen. In einer bevorzugten Ausführungsform weisen zwei gegenüberliegende schlauchförmige Kammern eine geringere Dicke auf als die übrigen Kammern. In einer Ausführungsform weist jede der schlauchförmigen Kammern eine geringere Dicke auf als die Zentralkammern

In einer Ausführungsform hat der Flaschenkörper in der Draufsicht die Form eines Rechteckes, einschließlich eines Quadrats, an dessen Kanten die Kammern angesetzt sind. In einer bevorzugten Ausführungsform sind die schlauchförmigen Kammern über die gesamte Länge mit den Kanten des rechteckigen Flaschenkörpers verbunden.

In einer vorteilhaften Ausführungsform sind sich am Flaschenkörper gegenüber angeordnete Kammern in der Draufsicht spiegelbildlich ausgebildet. So können sich die Paare gegenüberliegender schlauchförmiger Kammern ebenso in den Abmessungen und der Form auch unterscheiden.

In einer vorteilhaften Ausgestaltung der Erfindung entspricht in der Draufsicht die Fläche mindestens einer Kammer in ihren Abmessungen maximal der Fläche des Flaschenkörpers in der Draufsicht. Eine solche Kammer kann vollständig auf den Flaschenkörper geklappt werden, wodurch die effektive Wärme- bzw. Kältefläche der Wärmflasche um die Fläche der auf den Flaschenkörper geklappten Kammer verkleinert wird. Vorzugsweise entspricht die Fläche jeder Kammern in der Draufsicht maximal der Fläche des Flaschenkörpers.

Ebenso können zur weiteren Anpassung der Wärme- oder Kältefläche an die zu behandelnde Körperfläche die weiteren Kammern auf den Flaschenkörper geklappt werden, sodass die Wärme- oder Kälteflache der Wärmflasche die in direktem Kontakt mit der zu behandelnden Körperpartie steht, weiter minimiert wird. Falls alle vier Kammern auf den Flaschenkörper geklappt sind, entspricht die Wärmefläche der Wärmflasche der Fläche des Flaschenkörpers. Somit ermöglicht die erfindungsgemäße Wärmflasche sowohl eine großflächige als auch eine punktuelle Wärme- oder Kältebehandlung.

In einer vorteilhaften Ausgestaltung der Erfindung ist zumindest ein Verbindungsbereich zwischen Flaschenkörper und einer Kammer als Falz ausgebildet. Dieser Bereich weist somit eine geringere Dicke auf als der Flaschenkörper und die Kammer selbst, wodurch ein Umklappen der Kammer auf den Flaschenkörper erleichtert ist.

In einer vorteilhaften Ausgestaltung sind die Befestigungsmittel als Klettverschluss, Druckknopf oder Reißverschluss ausgebildet. Unter Befestigungsmittel sind ebenso Haftmittel beispielsweise in Form von Klebestreifen oder gummierten Bereichen zur besseren Haftung an Vlies und/oder Textilien zu verstehen.

In einer vorteilhaften Ausgestaltung der Erfindung umfasst die Wärmflasche mindestens eine verschließbare Einfüll- und Entleeröffnung zum Einfüllen und Entleeren des Wärmeübertragungsmediums. Die mindestens eine verschließbare Einfüll- und Entleeröffnungen kann entweder in einer der schlauchförmigen Kammern oder in dem Flaschenkörper angeordnet sein.

In einer vorteilhaften Ausgestaltung der Erfindung befindet sich die mindestens eine verschließbare Einfüll- und Entleeröffnung zum Einfüllen und Entleeren des Wärmeübertragungsmediums an dem der Zentralkammer entgegengesetzten Ende einer der schlauchförmigen Kammern. Durch diese Anordnung wird das vollständige Entleeren der Wärmflasche erleichtert, da die Wärmflasche so ausgerichtet werden kann, dass die Einfüll- und Entleeröffnung in Richtung des Bodens zeigt und somit den tiefsten Punkt einnimmt, sodass das Wärmeübertragungsmedium vollständig ausgelassen werden kann. Ebenso wird durch eine solche Anordnung der Einfüll- und Entleeröffnung das Befüllen erleichtert, da die Einfüll- und Entleeröffnung so ausgerichtet werden kann, dass sie den höchsten Punkt einnimmt, sodass die Luft beim Befüllen vollständig durch die Einfüll- und Entleeröffnung entweichen kann.

In einer vorteilhaften Ausgestaltung umfasst die Wärmflasche eine Einfüllöffnung zum Einfüllen des Wärmeübertragungsmediums und eine Entleeröffnung zum Entleeren des Wärmeübertragungsmediums, die jeweils in sich gegenüberliegenden Kammern angeordnet sind. Eine solche Wärmflasche kann an einen Wärmeübertragungsmediumsstrom, beispielsweise einen Warm- oder Kaltwasserstrom, angeschlossen werden, sodass das Wärmeübertragungsmedium durch die erste Einfüllöffnung in die Wärmflasche geleitet wird, sich in dieser verteilt und durch die Entleeröffnung wieder aus der Wärmflasche geleitet wird. Die Einfüllöffnung und die Entleeröffnung können über jeweils eine ventilgesicherte Zuführungs- bzw. Abführungsleitung mit einem Vorratsbehälter für das Wärmeübertragungsmedium in Fluidverbindung stehen. Die Fluidverbindung zwischen der Wärmflasche und dem Vorratsbehälter kann durch Schließen eines Ventils in der Zuführungs- bzw. Abführungsleitung vorrübergehend unterbrochen werden. Durch Öffnen des Ventils wird die Fluidverbindung wieder hergestellt und der Austausch des Wärmeübertragungsmediums in der Wärmflasche wird vorgesetzt.

In der erfindungsgemäßen Wärmflasche kann zudem zwischen der Zentralkammer zur Aufnahme eines Wärmeübertragungsmediums und mindestens einer der schlauchförmigen Kammern ein Absperrmittel angeordnet sein. Dieses Absperrmittel, bevorzugt in Form eines Ventils, kann das Ausbreiten des Wärmeübertragungsmediums zwischen der Zentralkammer und einer der schlauchförmigen Kammern verhindern. Auf diese Weise ist es möglich, die Füllung der abgesperrten Kammer mit Wärmeübertragungsmedium zu verhindern, sodass die nicht gefüllte Kammer eine geringere Dicke aufweist als die Zentralkammer. Hierdurch ist ein Umklappen auf die Zentralkammer erleichtert oder gar überflüssig, da die abgesperrte Kammer keine Wärme oder Kälte auf die Körperpartie, die nicht behandelt werden soll, überträgt.

In einer vorteilhaften Ausgestaltung der Erfindung umfasst die Wärmflasche einen Stoffüberzug, insbesondere einen abnehmbaren Stoffüberzug. Der Stoffüberzug minimiert die Gefahr von Verbrennungen und erzeugt ein angenehmes Tragegefühl auf der Haut. Im Falle eines abnehmbaren Stoffüberzuges kann dieser separat gereinigt werden.

In einer vorteilhaften Ausgestaltung der Erfindung umfasst die erfindungsgemäße Wärmflasche ein elektrisches Heizelement, bevorzugt in Form eines elektrischen Heizdrahtes. Auf diese Weise kann die Wärmflasche durch Anlegen einer Spannung dauerhaft warmgehalten werden.

Bevorzugt umfasst die erfindungsgemäße Wärmflasche mindestens zwei Elektroden sowie eine Stromquelle, bevorzugt eine Wechselstromquelle, zur Durchführung einer Elektrotherapie. Bei Körperkontakt der zwei Elektroden kann Strom von einer Elektrode über den Körper zur zweiten Elektrode fließen, sodass zeitgleich zur Wärme- bzw. Kältebehandlung, eine Elektrotherapie durchgeführt werden kann.

Die Erfindung wird im Folgenden anhand der Figuren erläutert. Dabei zeigen:
Figur 1 die Draufsicht einer Ausführungsform einer erfindungsgemäßen Wärmflasche.
Figur 2 eine dreidimensionale Ansicht einer Ausführungsform einer erfindungsgemäßen Wärmflasche.

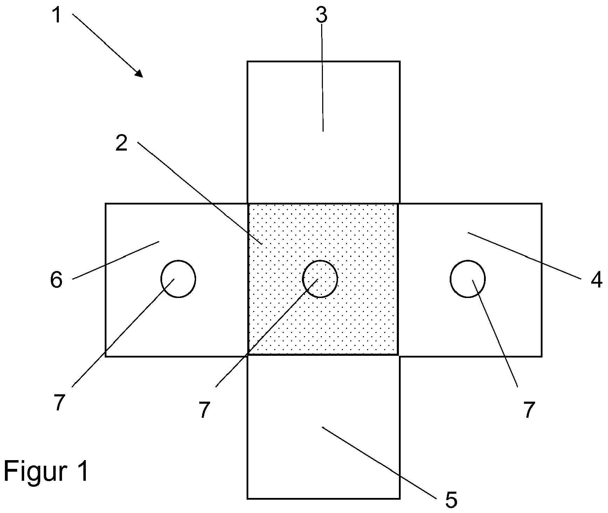

Figur 1 zeigt eine Ausführungsform einer erfindungsgemäßen Wärmflasche (1) mit einem Flaschenkörper (2) mit einer Zentralkammer zur Aufnahme eines Wärmeübertragungsmediums (gepunktet dargestellt). Die vier schlauchförmigen Kammern (3, 4, 5, 6) sind jeweils am Flaschenkörper (2) angesetzt und stehen in Fluidverbindung mit der Zentralkammer des Flaschenkörpers (2). Die vier schlauchförmigen Kammern (3, 4, 5, 6) sind an dem der Zentralkammer entgegengesetzten Enden verschlossen. Der Flaschenkörper (2) und die vier schlauchförmigen Kammern (3, 4, 5, 6) bilden in der Draufsicht die Form von Rechtecken aus, wobei die Fläche jeder Kammer (3, 4, 5, 6) maximal der Fläche des Flaschenkörpers (2) entspricht. Jede Kammer (3, 4, 5, 6) erstreckt sich über die gesamte Kantenlänge des Flaschenkörpers (2), an der sie angeordnet ist. Die vier Kammern (3, 4, 5, 6) bilden 90°-Winkel zwischen den Kammern aus. Jede Kammer (3, 4, 5, 6) kann vollständig auf den Flaschenkörper (2) geklappt werden, ohne diesen an den Seiten zu überragen. Auf diese Weise kann die effektive Behandlungsfläche angepasst werden. An dem Flaschenkörper (2) und den Kammern (4, 6) sind Befestigungsmittel (7) angeordnet. Diese Befestigungsmittel (7) können grundsätzlich zur Befestigung von einer schlauchförmigen Kammer und Zentralkammer aneinander oder an dem zu wärmenden/kühlenden Objekt, beispielsweise als Druckknopf oder Klettverschluss, ausgeführt sein. Es kann auch ein Verlängerungselement vorgesehen sein, an dem ebenfalls ein oder mehrere Befestigungselemente vorgesehen sein können, die mit einem oder mehreren Befestigungsmitteln an der Wärmflasche in Eingriff gebracht werden können.

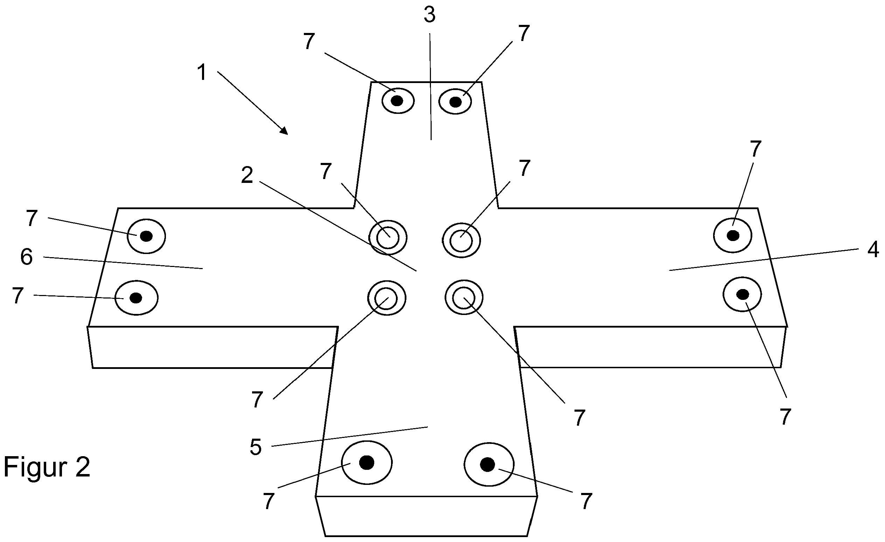

Figur 2 zeigt eine dreidimensionale Ansicht einer erfindungsgemäßen als Flachkörper ausgebildeten Wärmflasche (1). Die Kammern (3, 4, 5, 6) stehen mit dem Flaschenkörper (2) in Fluidverbindung, da sie gemeinsam eine einzige große Kammer ausbilden, in der sich das Wärmeübertragungsmedium verteilen kann. Das Wärmeübertragungsmedium kann durch eine verschließbare Einfüll- und Entleeröffnung (nicht gezeigt) eingefüllt und wieder ausgelassen werden. An den Kammern (3, 4, 5, 6) sind je zwei Befestigungsmittel angeordnet und an dem Flaschenkörper (2) sind vier Befestigungsmittel angeordnet. Die Befestigungsmittel (7) sind als Druckknöpfe ausgebildet, d.h. dass die Druckknopfhälften mit Zapfen an den Kammern angeordnet und die komplementären Druckknopfhälften an dem Flaschenkörper angeordnet sind. Ebenso ist es möglich, die Befestigungsmittel als Klett- oder Reißverschlüsse oder sonstige Haftmittel auszubilden.

### Bezugszeichenliste

(1) Wärmflasche
(2) Flaschenkörper
(3) schlauchförmige Kammer
(4) schlauchförmige Kammer
(5) schlauchförmige Kammer
(6) schlauchförmige Kammer
(7) Befestigungsmittel

## Patentansprüche

1. Wärmflasche (1) mit einem Flaschenkörper (2) mit einer Zentralkammer zur Aufnahme eines Wärmeübertragungsmediums und mit vier schlauchförmigen Kammern (3, 4, 5, 6), die jeweils am Flaschenkörper (2) über einen Verbindungsbereich angesetzt in Fluidverbindung mit der Zentralkammer im Flaschenkörper (2) stehen und die an dem der Zentralkammer entgegengesetzten Ende verschlossen sind, wobei die Wärmflasche (1) in der Draufsicht die Form eines Kreuzes hat, wobei an zumindest zwei von Flaschenkörper (2) und Kammern (3, 4, 5, 6) Befestigungsmittel (7) angeordnet sind.

2. Wärmflasche (1) nach Anspruch 1, wobei die Befestigungsmittel (7) zur Befestigung aneinander ausgebildet sind.

3. Wärmflasche (1) nach Anspruch 1 oder 2, wobei die Befestigungsmittel (7) zur Befestigung der Wärmflasche an dem zu wärmenden Objekt ausgebildet sind.

4. Wärmflasche (1) nach einem der vorhergehenden Ansprüche, wobei die Befestigungsmittel (7) zur Befestigung der Wärmflasche (1) an Textilien ausgebildet sind.

5. Wärmflasche (1) nach einem der vorhergehenden Ansprüche, wobei die Befestigungsmittel (7) am Flaschenkörper (2) und an jeder Kammer (3, 4, 5, 6) angeordnet sind.

6. Wärmflasche (1) nach einem der vorhergehenden Ansprüche, wobei die Wärmflasche zusätzlich ein Verlängerungselement, das über zumindest eines der Befestigungsmittel (7) mit dem Flaschenkörper oder einer der Kammern verbunden ist, umfasst.

7. Wärmflasche (1) nach einem der vorhergehenden Ansprüche, wobei sich am Flaschenkörper (2) gegenüber angeordnete Kammern (3, 5 bzw. 4, 6) in der Draufsicht spiegelbildlich ausgebildet sind.

8. Wärmflasche (1) nach einem der vorhergehenden Ansprüche, wobei der Flaschenkörper (2) in der Draufsicht die Form eines Rechteckes hat, an dessen Kanten die Kammern (3, 4, 5, 6), vorzugsweise über die gesamte Länge der Kante, angesetzt sind.

9. Wärmflasche (1) nach einem der vorhergehenden Ansprüche, wobei die Fläche mindestens einer Kammer (3, 4, 5, 6) in der Draufsicht maximal der Fläche des Flaschenkörpers (2) in der Draufsicht entspricht.

10. Wärmflasche (1) nach einem der vorhergehenden Ansprüche, wobei zumindest ein Verbindungsbereich zwischen Flaschenkörper (2) und einer Kammer (3, 4, 5, 6) als Falz ausgebildet ist.

11. Wärmflasche (1) nach einem der vorhergehenden Ansprüche, wobei die Befestigungsmittel (7) als Klettverschluss, Druckknopf oder Reißverschluss ausgebildet sind.

12. Wärmflasche (1) nach einem der vorhergehenden Ansprüche mit mindestens einer verschließbaren Einfüll- und Entleeröffnung zum Einfüllen und Entleeren des Wärmeübertragungsmediums.

13. Wärmflasche (1) nach einem der vorhergehenden Ansprüche mit einem Schutzüberzug, insbesondere einem abnehmbaren Stoffüberzug.

14. Wärmflasche (1) nach einem der vorhergehenden Ansprüche, umfassend ein elektrisches Heizelement, bevorzugt in Form eines elektrischen Heizdrahtes.

15. Wärmflasche (1) nach einem der vorhergehenden Ansprüche umfassend mindestens zwei Elektroden sowie eine Stromquelle, bevorzugt eine Wechselstromquelle, zur Durchführung einer Elektrotherapie.
